(19) Europäisches Patentamt
European Patent Office
Office européen des brevets

(11) EP 2 065 074 B1

(12) **FASCICULE DE BREVET EUROPEEN**

(45) Date de publication et mention de la délivrance du brevet:
**18.06.2014 Bulletin 2014/25**

(51) Int Cl.:
***A61Q 5/10** (2006.01)* ***A61K 8/73** (2006.01)*
***A61K 8/81** (2006.01)*

(21) Numéro de dépôt: **08168646.1**

(22) Date de dépôt: **07.11.2008**

(54) **Composition de teinture d'oxydation des fibres kératiniques comprenant une cellulose à substituant(s) hydrophobe(s), un colorant d'oxydation et un polymère cationique**

Zusammensetzung zum Oxidationsfärben von Keratinfasern enthaltend eine Cellulose mit hydrophobischen Gruppe, ein Oxidationsfarbmittel und ein kationisches Polymer

Composition for the oxidation dyeing of keratin fibres comprising a cellulose with hydrophobic substituent (s), an oxidation dye and a cationic polymer

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **09.11.2007 FR 0758931**

(43) Date de publication de la demande:
**03.06.2009 Bulletin 2009/23**

(73) Titulaire: **L'Oréal**
**75008 Paris (FR)**

(72) Inventeurs:
- **Audousset, Marie-Pascale**
**92600 Asnieres (FR)**
- **Ascione, Jean-Marc**
**75003 Paris (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 426 040** **EP-A- 1 707 190**
**WO-A-02/45674** **WO-A-98/03150**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente demande a pour objet une composition de teinture d'oxydation des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences.

Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (i.e. abîmées) de sa pointe à sa racine.

Par ailleurs, les compositions obtenues doivent, en outre, présenter de bonnes propriétés rhéologiques, tout en conservant de bonnes propriétés de coloration. En particulier, ces compositions ne doivent pas couler sur le visage ou en dehors des zones que l'on se propose de teindre, lors de leur application, notamment après mélange avec un agent oxydant.

Il est déjà connu de la demande WO 98/03150 d'améliorer la puissance de la coloration en associant une base d'oxydation para-phénylènediamine et au moins un polymère amphiphile non ionique du type hydroxycellulose modifiée par un groupement hydrophobe.

Il est aussi connu du document EP 1 707 190 une composition de coloration des fibres kératiniques qui comprend une hydroxy cellulose alkylée en C14/C16, des colorants d'oxydation et du polyquaternium-6.

Cependant, ces compositions ne satisfont pas pleinement les exigences précitées et peuvent être améliorées, notamment en termes de propriétés tinctoriales, en particulier au niveau de la sélectivité et de la puissance de coloration. Le but de la présente invention est l'obtention de compositions de coloration capillaire stables, notamment sous forme de crèmes, faciles à préparer et à appliquer, pouvant contenir des concentrations élevées de colorants sous forme de sels, ayant de bonnes qualités rhéologiques et conduisant à des colorations intenses, peu sélectives et résistantes aux diverses agressions que peuvent subir les fibres kératiniques.

Ce but est atteint par la présente invention qui a pour objet une composition tinctoriale pour fibres kératiniques, et en particulier pour fibres kératiniques humaines telles que les cheveux, comprenant, dans un milieu approprié pour la teinture :

(A) un ou plusieurs dérivé(s) non ionique(s) de cellulose comprenant un ou plusieurs substituant(s) hydrophobe(s) comprenant de 8 à 30 atomes de carbone,

(B) un ou plusieurs colorants d'oxydation, et

(C) un ou plusieurs polymères cationiques diammonium quaternaire contenant des motifs récurrents répondant à la formule (I) :

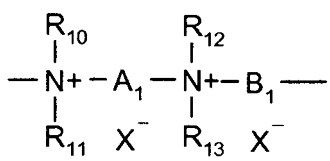

formule dans laquelle :

$R_{10}$, $R_{11}$, $R_{12}$ et $R_{13}$, identiques ou différents, représentent des radicaux aliphatiques, alicycliques, ou arylaliphatiques en $C_1$-$C_{20}$ ou des radicaux hydroxyalkylaliphatiques dont le radical alkyle est en $C_1$-$C_4$, ou bien $R_{10}$, $R_{11}$, $R_{12}$ et

$R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH-$R_{14}$-D où $R_{14}$ est un alkylène et D un groupement ammonium quaternaire ;
$A_1$ et $B_1$ représentent des groupements polyméthyléniques en $C_2$-$C_{20}$, linéaires ou ramifiés, saturés ou non, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X- désigne un anion dérivé d'un acide minéral ou organique ;
$A_1$, $R_{10}$ et $R_{12}$ peuvent former avec les deux atomes d'azote auxquels ils sont rattachés un cycle pipérazinique ;

en outre si $A_1$ désigne un radical alkylène ou hydroxyalkylène linéaire ou ramifié, saturé ou insaturé, $B_1$ peut également désigner un groupement $-(CH_2)_n$-CO-D-OC-$(CH_2)_n$- dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes : $-(CH_2-CH_2-O)_x-CH_2-CH_2-$ ; $-[CH_2-CH(CH_3)-O]_y-CH_2-CH(CH_3)-$ où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical $-CH_2-CH_2-S-S-CH_2-CH_2-$ ;
d) un groupement uréylène de formule : -NH-CO-NH-.

Les compositions tinctoriales selon l'invention présentent notamment les propriétés suivantes :

- elles permettent d'obtenir des compositions de viscosité correspondant à une crème qui sont stables dans le temps,
- elles se distinguent par une facilité de mélange avec la composition oxydante,
- elles se distinguent par les qualités rhéologiques des crèmes obtenues (bonne viscosité de crème en mélange),
- elles sont faciles à appliquer après mélange avec la composition oxydante au moment de la mise en oeuvre de la coloration (qualités d'usage sur tête).

En outre, les compositions selon l'invention permettent l'obtention de compositions capables de conduire à des colorations aux nuances variées, chromatiques, puissantes, esthétiques, peu sélectives, uniformes sur l'ensemble des fibres kératiniques et en particulier des fibres kératiniques humaines telles que les cheveux, et résistant bien aux diverses agressions que peuvent subir les fibres.
Un autre objet de la présente invention consiste en un procédé de teinture des fibres kératiniques dans lequel la composition cosmétique selon l'invention est mise en oeuvre.
Un troisième objet de l'invention concerne l'utilisation de cette composition cosmétique pour la teinture des fibres kératiniques, et en particulier des fibres kératiniques humaines, telles que les cheveux.
D'autres caractéristiques, aspects, objets et avantages de la présente invention apparaîtront encore plus clairement à la lecture de la description et des exemples qui suivent.
A moins d'une indication différente, les bornes des gammes de valeurs qui sont données dans le cadre de la présente invention sont incluses dans ces gammes.
Par « dérivé(s) de cellulose », on entend un (ou des) composé(s) comportant au moins un motif cellobiose de structure suivante :

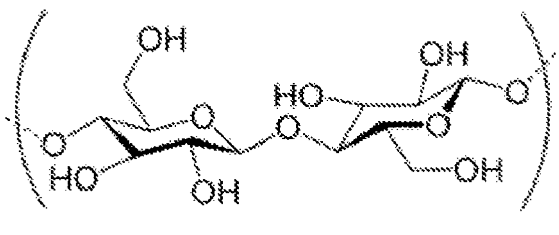

dans laquelle, un ou plusieurs groupe(s) hydroxyle peut (ou peuvent) être substitué(s).
Le ou les dérivés non ioniques de cellulose à substituant(s) hydrophobe(s) (A) conformes à la présente invention,

sont des polymères amphiphiles présentant un caractère associatif. En effet, ils comprennent des motifs hydrophiles et des motifs hydrophobes et sont capables d'interagir et de s'associer entre eux ou à d'autres molécules, de manière réversible, en particulier, grâce à la présence de leurs chaînes hydrophobes.

De préférence, le dérivé de cellulose de l'invention est un éther de cellulose comprenant un ou plusieurs substituant(s) hydrophobe(s) comprenant de 8 à 30 atomes de carbone.

Le ou les dérivés non ioniques de cellulose à substituant(s) hydrophobe(s) conformes à la présente invention, sont préparés généralement à partir d'éthers non-ioniques de cellulose hydrosolubles, dont on substitue tout ou partie des fonctions hydroxyle réactives par une ou plusieurs chaîne(s) hydrophobe(s) comprenant de 8 à 30 atomes de carbone, de préférence de 10 à 22 atomes de carbone, et mieux encore 16 atomes de carbone. Les étapes de réactions en jeu dans la préparation des dérivés de cellulose de l'invention sont connues de l'homme du métier.

Les éthers non-ioniques de cellulose choisis pour préparer les dérivés non-ioniques de cellulose à substituant(s) hydrophobe(s) selon l'invention, ont de préférence un degré de substitution non-ionique, par exemple en groupement(s) méthyle, hydroxyéthyle ou hydroxypropyle, suffisant pour être hydrosolubles, c'est-à-dire former une solution sensiblement limpide lorsqu'ils sont dissous dans l'eau à 25 °C à la concentration de 1 % en poids.

Les éthers non ioniques de cellulose choisis pour préparer les dérivés non ioniques de cellulose à substituant(s) hydrophobe(s) selon l'invention, possèdent préférentiellement une masse molaire moyenne en nombre relativement faible, inférieure à 800 000 g/mole, de préférence allant de 50 000 et 700 000 g/mole et de manière plus préférée allant de 200 000 à 600 000 g/mole.

De préférence, le dérivé de cellulose de l'invention est une hydroxyéthylcellulose comprenant un ou plusieurs substituant(s) hydrophobe(s) comprenant de 8 à 30 atomes de carbone.

Les dérivés non-ioniques de cellulose utilisés selon l'invention sont substitués, par une ou plusieurs chaîne(s) hydrocarbonée(s) en $C_8$-$C_{30}$ linéaires, ramifiées ou cycliques, saturées ou insaturées, aliphatiques ou aromatiques, pouvant être rattachées au substrat éther de cellulose par une liaison éther, ester, ou uréthane, de préférence éther.

Selon un mode de réalisation, le ou les substituants hydrophobes utilisés comme substituants des dérivés non-ioniques de cellulose selon la présente invention sont des groupes alkyle, arylalkyle ou alkylaryle en $C_8$-$C_{30}$, de préférence en $C_{10}$-$C_{22}$.

De préférence, le ou les substituants hydrophobes selon la présente invention sont des chaînes alkyles saturées.

Selon un mode de réalisation préféré, le ou les substituants hydrophobes selon la présente invention sont des groupements cétyle.

Les dérivés non-ioniques de cellulose à substituant(s) hydrophobe(s) selon l'invention, présentent une viscosité de préférence comprise entre 100 et 100 000 mPa.s, et de préférence entre 200 et 20 000 mPa.s, mesurée à 25 °C dans une solution à 1 % en poids de polymère dans l'eau, cette viscosité étant déterminée de manière conventionnelle à l'aide d'un viscosimètre de type Brookfield LVT à 6 tours par minute avec le mobile n° 3.

Le degré de substitution hydrophobe des dérivés non-ioniques de cellulose hydrophiles utilisés selon l'invention, va préférentiellement de 0,1 à 10 % en poids, plus préférentiellement de 0,1 à 1 % en poids et de manière particulièrement préférée de 0,4 à 0,8 % en poids, du poids total du polymère.

Parmi les dérivés non-ioniques de cellulose à substituant(s) hydrophobe(s) utilisables dans les compositions de l'invention, on peut citer, de préférence, les cétyl hydroxyéthylcelluloses commercialisées sous les dénominations Natrosol Plus Grade 330 CS et Polysurf 67 CS (INCI : Cetyl Hydroxyethylcellulose) par la société Aqualon/Hercules.

La concentration en dérivé(s) non-ionique(s) de cellulose à substituant(s) hydrophobe(s) (A) des compositions selon l'invention va de préférence de 0,01 à 10 % en poids, en particulier de 0,05 à 3 % en poids, et de manière plus préférée de 0,1 à 1 % en poids, par rapport au poids total de la composition.

La ou les colorants d'oxydation utiles dans la présente invention peuvent être choisis parmi les bases d'oxydation et les coupleurs.

Le (ou les) colorant(s) d'oxydation (C) utilisable(s) selon l'invention est (ou sont) de préférence choisi(s) parmi les bases d'oxydation, les coupleurs d'oxydation, et leurs sels d'addition.

A titre d'exemple, les bases d'oxydation utilisables sont choisies parmi les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N diéthyl 3-méthyl aniline, la N,N-bis-(β-hydroxyéthyl) para-phénylènediamine, la 4-N,N-bis-(β-hydroxyéthyl)amino-2-méthyl aniline, la 4 N,N bis (β-hydroxyéthyl)amino-2-chloro aniline, la 2-β-hydroxyéthyl para-phénylènediamine, la 2-fluoro-para-phénylènediamine, la 2-isopropyl para-phénylènediamine, la N (2'-hydroxypropyl) para-phénylènediamine, la 2-hydroxyméthyl para-phénylènediamine, la N,N diméthyl 3-méthyl para-phénylènediamine, la N,N (éthyl, 2'-hydroxyéthyl) para-phénylènediamine, la N-(β,γ-dihydroxypropyl) para-phénylènediamine, la N (4' aminophényl) para-phénylènediamine,

la N phényl para-phénylènediamine, la 2 (2'-hydroxyéthyloxy)para-phénylènediamine, la 2-β-acétylaminoéthyloxy para-phénylènediamine, la N (β-méthoxyéthyl) para-phénylènediamine, la 4-aminophénylpyrrolidine, la 2-thiényl para-phénylènediamine, le 2-β hydroxyéthylamino-5-aminotoluène, la 3-hydroxy-1-(4'-aminophényl)pyrrolidine et leurs sels d'addition avec un acide.

Parmi les para-phénylènediamines citées ci-dessus, la para-phénylènediamine, la para-toluylènediamine, la 2 isopropyl para-phénylènediamine, la 2-β-hydroxyéthyl para-phénylènediamine, la 2 β hydroxyéthyloxy para-phénylènediamine, la 2,6-diméthyl para-phénylènediamine, la 2,6-diéthyl para-phénylènediamine, la 2,3 diméthyl para-phénylènediamine, la N,N bis-(β-hydroxyéthyl) para-phénylènediamine, la 2 chloro para-phénylènediamine, la 2 β acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3 diamino propanol, la N,N'-bis-(β-hydroxyéthyl) N,N'-bis-(4'-aminophényl) éthylènediamine, la N,N' bis-(4-aminophényl) tétraméthylènediamine, la N,N' bis (β hydroxyéthyl) N,N'-bis-(4-aminophényl) tétraméthylènediamine, la N,N' bis-(4-méthyl-aminophényl) tétraméthylènediamine, la N,N'-bis-(éthyl) N,N' bis (4'-amino, 3' méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino-3-méthyl phénol, le 4 amino-3 fluoro phénol, le 4 amino-3-hydroxyméthyl phénol, le 4-amino-2-méthyl phénol, le 4-amino-2 hydroxyméthyl phénol, le 4-amino-2-méthoxyméthyl phénol, le 4 amino-2 aminométhyl phénol, le 4-amino-2-(β-hydroxyéthyl aminométhyl)phénol, le 4 amino 2-fluoro phénol, et leurs sels d'addition avec un acide.

Parmi les ortho-aminophénols, on peut citer à titre d'exemple, le 2-aminophénol, le 2-amino-5-méthylphénol, le 2-amino-6 méthylphénol, le 5-acétamido-2 aminophénol, et leurs sels d'addition avec un acide.

Parmi les bases hétérocycliques, on peut citer à titre d'exemple, les dérivés pyridiniques, les dérivés pyrimidiniques, les dérivés pyrazoliques, les dérivés de pyrazolone, et leurs sels d'addition.

Parmi les dérivés pyridiniques, on peut citer les composés décrits par exemple dans les brevets GB 1026978 et GB 1153196, comme la 2,5 diamino pyridine, la 2-(4-méthoxyphényl)amino 3 amino pyridine, la 2,3 diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5 a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88 169571 ; JP 05 63124 ; EP 0770375 ou demande de brevet WO 96/15765 comme la 2,4,5,6-tétra-aminopyrimidine, la 4 hydroxy 2,5,6-triaminopyrimidine, la 2 hydroxy-4,5,6-triaminopyrimidine, la 2,4 dihydroxy-5,6-diaminopyrimidine, la 2,5,6 triaminopyrimidine, et les dérivés pyrazolopyrimidiniques tels ceux mentionnés dans la demande de brevet FR A 2750048 et parmi lesquels on peut citer la pyrazolo-[1,5-a]-pyrimidine-3,7-diamine ; la 2,5-diméthyl pyrazolo [1,5 a]-pyrimidine-3,7-diamine ; la pyrazolo-[1,5-a]-pyrimidine-3,5-diamine ; la 2,7 diméthyl pyrazolo-[1,5-a]-pyrimidine-3,5-diamine ; le 3-amino pyrazolo-[1,5 a]-pyrimidin-7-ol ; le 3-amino pyrazolo-[1,5-a]-pyrimidin-5-ol ; le 2 (3 amino pyrazolo-[1,5-a]-pyrimidin-7-ylamino)-éthanol, le 2 (7-amino pyrazolo-[1,5-a]-pyrimidin-3-ylamino)-éthanol, le 2-[(3-amino-pyrazolo[1,5-a]pyrimidin-7-yl)-(2 hydroxy-éthyl)-amino]-éthanol, le 2-[(7-amino-pyrazolo[1,5-a]pyrimidin-3-yl)-(2 hydroxy-éthyl)-amino]-éthanol, la 5,6-diméthyl pyrazolo-[1,5-a]-pyrimidine-3,7 diamine, la 2,6 diméthyl pyrazolo-[1,5-a]-pyrimidine-3,7-diamine, la 2, 5, N7, N7-tétraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques utilisables, on peut citer par exemple les composés décrits dans les brevets DE A 38 43 892, DE A 41 33 957 et demandes de brevet WO 94/08969, WO 94/08970, FR A-2 733 749 et DE A 195 43 988 comme le 4,5 diamino-1-méthylpyrazole, le 4,5-diamino-1-(β-hydroxyéthyl)pyrazole, le 3,4 diaminopyrazole, le 4,5-diamino-1 (4' chlorobenzyl)pyrazole, le 4,5-diamino-1,3-diméthylpyrazole, le 4,5 diamino-3-méthyl-1-phényl-pyrazole, le 4,5-diamino-1-méthyl-3-phénylpyrazole, le 4-amino-1,3-diméthyl-5-hydrazino-pyrazole, le 1 benzyl-4,5-diamino-3-méthylpyrazole, le 4,5-diamino-3-tert-butyl-1-méthylpyrazole, le 4,5-diamino-1-tert-butyl-3-méthyl-pyrazole, le 4,5-diamino-1 (β hydroxyéthyl)-3-méthylpyrazole, le 4,5 diamino-1 éthyl-3-méthylpyrazole, le 4,5-diamino-1-éthyl-3 (4' méthoxyphényl)-pyrazole, le 4,5-diamino-1-éthyl-3 hydroxy-méthyl-pyrazole, le 4,5-diamino-3-hydroxyméthyl-1 méthyl-pyrazole, le 4,5-diamino-3 hydroxyméthyl-1-isopropylpyrazole, le 4,5 diamino-3-méthyl-1 isopropylpyrazole, le 4-amino-5-(2'-aminoéthyl)amino-1,3 diméthyl-pyrazole, le 3,4,5 triaminopyrazole, le 1-méthyl-3,4,5-triaminopyrazole, le 3,5-diamino-1 méthyl-4-méthylaminopyrazole, le 3,5-diamino-4 (β hydroxyéthyl)amino-1 méthylpyrazole, et leurs sels d'addition.

Parmi les dérivés de pyrazolone utilisables, on peut citer par exemple les composés suivants et leurs sels d'addition :

2,3-diaminodihydropyrazolone ;
4,5-diamino-1,2-diméthyl-1,2-dihydropyrazol-3-one ;
4-amino-5-méthylamino-1,2-diméthyl-1,2-dihydropyrazol-3-one ;
4-amino-5-diméthylamino-1,2-diméthyl-1,2-dihydropyrazol-3-one ;
4-amino-5-(2-hydroxyéthyl)amino-1,2-diméthyl-1,2-dihydropyrazol-3-one ;
4-amino-5-(pyrrolidin-1-yl)-1,2-diméthyl-1,2-dihydropyrazol-3-one ;
4-amino-5-(pipéridin-1-yl)-1,2-diméthyl-1,2-dihydropyrazol-3-one ;
4,5-diamino-1,2-di-(2-hydroxyéthyl)-1,2-dihydropyrazol-3-one ;
4-amino-5-méthylamino-1,2-di-(2-hydroxyéthyl)-1,2-dihydropyrazol-3-one ;
4-amino-5-diméthylamino-1,2-di-(2-hydroxyéthyl)-1,2-dihydropyrazol-3-one ;
4-amino-5-(2-hydroxyéthyl)amino-1 ,2-di-(2-hydroxyéthyl)-1 ,2-dihydropyrazol-3-one ;
4-amino-5-(pyrrolidin-1-yl)-1,2-di-(2-hydroxyéthyl)-1,2-dihydropyrazol-3-one ;
4-amino-5-(pipéridin-1-yl)-1,2-di-(2-hydroxyéthyl)-1,2-dihydropyrazol-3-one ;
4,5-diamino-1,2-diéthyl-1,2-dihydropyrazol-3-one ;
4,5-diamino-1,2-diphényl-1,2-dihydropyrazol-3-one ;
4,5-diamino-1-éthyl-2-méthyl-1,2-dihydropyrazol-3-one ;
4,5-diamino-2-éthyl-1-méthyl-1,2-dihydropyrazol-3-one ;
4,5-diamino-1-phényl-2-méthyl-1,2-dihydropyrazol-3-one ;
4,5-diamino-2-phényl-1-méthyl-1,2-dihydropyrazol-3-one ;
4,5-diamino-1-(2-hydroxyéthyl)-2-méthyl-1,2-dihydropyrazol-3-one ;
4,5-diamino-2-(2-hydroxyéthyl)-1-méthyl-1,2-dihydropyrazol-3-one ;
2,3-diamino-6,7-dihydro-1H,5H-pyrazolo[1,2-a]pyrazol-1-one ;
2-amino-3-méthylamino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one ;
2-amino-3-éthylamino-6,7-dihydro-1H,5H-pyrazolo[1,2-a]pyrazol-1-one ;
2-amino-3-isopropylamino-6,7-dihydro-1H,5H-pyrazolo[1,2-a]pyrazol-1-one ;
2-amino-3-(2-hydroxyéthyl)amino-6,7-dihydro-1H,5H-pyrazolo[1,2 a]-pyrazol-1-one ;
2-amino-3-(2-hydroxypropyl)amino-6,7-dihydro-1H,5H-pyrazolo[1,2-a]-pyrazol-1-one;
2-amino-3-bis(2-hydroxyéthyl)amino-6,7-dihydro-1H,5H-pyrazolo[1,2-a]-pyrazol-1-one;
2-amino-3-(pyrrolidin-1-yl)-6,7-dihydro-1H,5H-pyrazolo[1,2-a]pyrazol-1-one ;
2-amino-3-(3-hydroxy-pyrrolidin-1-yl)-6,7-dihydro-1H,5H-pyrazolo[1,2-a]-pyrazol-1-one ;
2-amino-3-(pipéridin-1-yl)-6,7-dihydro-1H,5H-pyrazolo[1,2-a]pyrazol-1-one ;
2,3-diamino-6-hydroxy-6,7-dihydro-1H,5H-pyrazolo[1,2-a]pyrazol-1-one ;
2,3-diamino-6-méthyl-6,7-dihydro-1H,SH-pyrazolo[1,2-a]pyrazol-1-one ;
2,3-diamino-6,6-diméthyl-6,7-dihydro-1H,5H-pyrazolo[1,2-a]pyrazol-1-one ;
2,3-diamino-5,6,7,8-tétrahydro-1H,6H-pyridazino[1,2-a]pyrazol-1-one ;
2,3-diamino-5,8-dihydro-1H,6H-pyridazino[1,2-a]pyrazol-1-one ;
4-amino-5-diméthylamino-1 ,2-diéthyl-1 ,2-dihydropyrazol-3-one ;
4-amino-1,2-diéthyl-5-éthylamino-1,2-dihydropyrazol-3-one ;
4- amino -1 ,2-diéthyl-5-isopropylamino-1 ,2-dihydropyrazol-3-one ;
4- amino -1,2-diéthyl-5-(2-hydroxyéthylamino)-1,2-dihydropyrazol-3-one ;
4-amino-5-(2-diméthylaminoéthylamino)-1,2-diéthyl-1,2-dihydropyrazol-3-one ;
4-amino-5-[bis(2-hydroxyéthyl)amino]-1,2-diéthyl-1,2-dihydropyrazol-3-one ;
4-amino-1,2-diéthyl-5-(3-imidazol-1-yl-propylamino)-1,2-dihydropyrazol-3-one ;
4-amino-1.2-diéthyl-5-(3-hydroxypyrrolidin-1-yl)-1,2-dihydropyrazol-3-one ;
4-amino-5-pyrrolidin-1-yl-1,2-diéthyl-1,2-dihydropyrazol-3-one ;
4-amino-5-(3-diméthylaminopyrrolidin-1-yl)-1,2-diéthyl-1,2-dihydropyrazol-3-one ;
4-amino-1,2-diéthyl-5-(4-méthylpipérazin-1-yl)pyrazolidin-3-one.

La concentration en base(s) d'oxydation va en général de 0,001 à 20% en poids, de préférence de 0,005 à 10 % en poids, et encore plus préférentiellement de 0,01 à 5% en poids, par rapport au poids total de la composition.

Le (ou les) coupleur(s) d'oxydation présent(s) dans les compositions de l'invention, peut (ou peuvent) être choisi(s) parmi les coupleurs benzéniques, les coupleurs hétérocycliques, les coupleurs naphtaléniques, et leurs sels d'addition.

A titre de coupleurs benzéniques utilisables dans les compositions selon l'invention, on peut citer les méta-aminophénols, les méta-phénylènediamines, les méta-diphénols, ainsi que leurs sels d'addition.

Parmi les coupleurs préférés, on peut citer le 2-méthyl-5-aminophénol, le 5-N-(β-hydroxyéthyl)amino-2-méthyl phénol, le 6-chloro-2-méthyl-5-aminophénol, le 3-aminophénol, le 1,3-dihydroxy benzène, le 1,3-dihydroxy-2-méthyl benzène, le 4-chloro-1,3-dihydroxy benzène, le 2,4-diamino-1-(f1-hydroxyéthyloxy)benzène, le 2-amino-4-(f1-hydroxyéthylamino)-1-méthoxybenzène, le 1,3-diamino benzène, le 1,3-bis-(2,4-diaminophénoxy)propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

De préférence, la composition de l'invention contient une ou plusieurs bases d'oxydation et un ou plusieurs coupleurs.

La concentration en coupleur(s) d'oxydation va en général de 0,001 à 20% en poids, de préférence de 0,005 à 10% en poids, et encore plus préférentiellement de 0,01 à 5% en poids, par rapport au poids total de la composition.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition comprend de plus au moins un polymère cationique.

Il est rappelé qu'au sens de la présente invention, l'expression "polymère cationique" désigne tout polymère contenant des groupements cationiques et/ou des groupements ionisables en groupements cationiques.

De préférence, la densité de charge cationique des polymères cationique selon l'invention est supérieure à 1 meq/g.

Cette densité de charge est déterminée par la méthode de Kjeldahl. Elle peut aussi se calculer à partir de la nature chimique du polymère. Dans la formule (I), de préférence, $X^-$ est un anion tel que le chlorure ou le bromure.

Ces polymères ont de préférence une masse moléculaire moyenne en nombre généralement comprise entre 1000 et 100000.

Des polymers de ce type sont notamment décrits dans FR 2320330, FR 2270846, FR 2316271, FR 2336434, FR 2413907, US 2273780, US 2375853, US 2388614, US 2454547, US 3206462, US 2261002, US 2271378, US 3874870, US 4001432, US 3929990, US 3966904, US 4005193, US 4025617, US 4025627, US 4025653, US 4026945 et US 4027020.

On peut utiliser plus particulièrement les polymères qui sont constitués de motifs récurrents répondant à la formule (II) suivante :

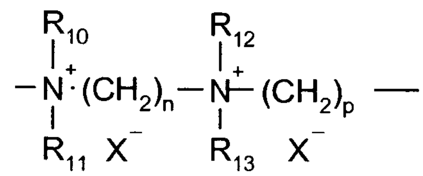

dans laquelle $R_{10}$, $R_{11}$, $R_{12}$ et $R_{13}$, identiques ou différents, désignent un radical alkyle ou hydroxyalkyle en $C_1$-$C_4$, n et p sont des nombres entiers variant de 2 à 20 environ et, $X^-$ est un anion dérivé d'un acide minéral ou organique.

Selon un mode particulier de l'invention, on utilise les polymères aux motifs récurrents de formules (W) et (U) suivantes :

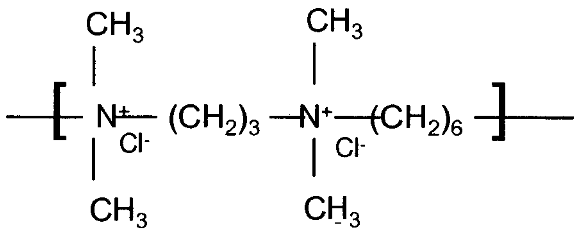

**(W)**

et notamment ceux dont le poids moléculaire, déterminé par chromatographie par perméation de gel, est compris entre 9500 et 9900 ;

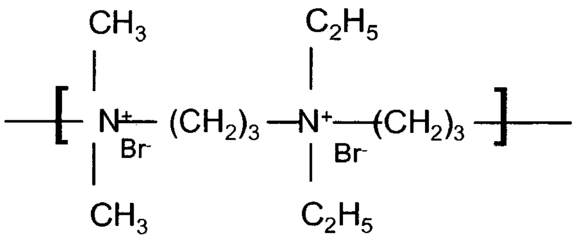

**(U)**

et notamment ceux dont le poids moléculaire, déterminé par chromatographie par perméation de gel, est d'environ 1200.

La concentration en polymères cationiques dans la composition selon la présente invention peut varier de 0,01 à 10% en poids par rapport au poids de la composition, de préférence de 0,05 à 6%, de préférence entre 0,1 et 5% en poids par rapport au poids de la composition.

La composition de l'invention peut contenir une ou plusieurs amide(s) d'acide gras. Le ou les amide(s) d'acide gras sont de préférence choisis parmi les amides d'une alcanolamine en $C_2$-$C_{10}$ et d'un acide gras en $C_{14}$-$C_{30}$, et encore plus préférentiellement parmi les amides d'une alcanolamine en $C_2$-$C_6$ et d'un acide gras en $C_{14}$-$C_{22}$.

Le ou les amide(s) d'acide gras sont généralement non ioniques, c'est-à-dire qu'elles ne comportent pas de charges ioniques.

L'amide d'une alcanolamine et d'un acide gras en $C_{14}$-$C_{30}$ est de préférence choisi parmi :

- le diéthanolamide d'acide oléique, tel que l'amide commercialisé sous la dénomination commerciale MEXANYL® GT par la société CHIMEX,
- le monoéthanolamide d'acide myristique, tel que l'amide commercialisé sous la dénomination commerciale COMPERLAN® MM par la société COGNIS,
- le diéthanolamide d'acides gras de soja, tel que l'amide commercialisé sous la dénomination commerciale COMPERLAN® VOD par la société COGNIS,
- l'éthanolamide d'acide stéarique, tel que l'amide commercialisé sous la dénomination commerciale MONAMID® S par la société UNIQEMA,
- le monoisopropanolamide d'acide oléique, tel que l'amide commercialisé sous la dénomination commerciale WITCAMIDE® 61 par la société WITCO,
- le diéthanolamide d'acide linoléique, tel que l'amide commercialisé sous la dénomination commerciale PURTON® SFD par la société ZSCHIMMER SCHWARZ,
- le monoéthanolamide d'acide stéarique, tel que l'amide commercialisé sous la dénomination commerciale MONAMID® 972 par la société ICI/UNIQEMA,
- le monoéthanolamide d'acide béhénique, tel que l'amide commercialisée sous la dénomination commerciale INCROMIDE® BEM de CRODA,
- le monoisopropanolamide d'acide isostéarique, tel que l'amide commercialisé sous la dénomination commerciale WITCAMIDE® SPA par la société WITCO,
- le diéthanolamide d'acide érucique, tel que l'amide commercialisé sous la dénomination commerciale diéthanolamide d'acide érucique par la société STEARINERIES DUBOIS,
- le monoéthanolamide d'acide ricinoléique, tel que l'amide commercialisé sous la dénomination commerciale monoéthanolamide ricinoléique par la société STEARINERIES DUBOIS,

La concentration en amide(s) d'acide gras des compositions selon l'invention va de préférence de 0 à 10%, en particulier de 0,2 à 10% en poids, et de manière plus préférée de 0,5 à 6% en poids, par rapport au poids total de la

composition.

La composition tinctoriale conforme à l'invention peut, en outre, contenir un ou plusieurs colorant(s) direct(s) pouvant notamment être choisi(s) parmi les colorants nitrés benzéniques, les colorants directs azoïques, les colorants directs méthiniques, les colorants anthraquinoniques, les colorants xanthéniques, les colorants triarylméthaniques et leurs sels d'addition. Ces colorants directs peuvent être de nature non ionique, anionique ou cationique.

Le milieu utilisé dans les compositions selon la présente invention est un milieu aqueux ou un milieu contenant de l'eau et au moins un solvant organique.

Le (ou les) solvant(s) organique(s) utilisé(s) dans les compositions selon la présente invention peut (ou peuvent) être choisi(s) parmi les alcools monohydroxylés et les polyols.

A titre d'alcools monohydroxylés utilisables, on peut citer les alcools inférieurs en $C_1$-$C_4$ comme l'éthanol, l'isopropanol, le tertiobutanol, le n-butanol, et leurs mélanges. De préférence, l'alcool utilisé est l'éthanol.

À titre de polyols utilisables, on peut citer le propylèneglycol, les polyéthylèneglycols, la glycérine. A titre de solvants organiques, on peut aussi citer les éthers de polyols comme le 2-butoxyéthanol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La concentration en solvant(s) organique(s) dans les compositions selon la présente invention est comprise de préférence entre 0 et 30%, et de manière plus préférée entre 0 et 20% en poids par rapport au poids total de la composition.

Les compositions selon la présente demande peuvent également contenir, un ou plusieurs agent(s) épaississant(s) additionnel(s)encore appelé "agent(s) d'ajustement de la rhéologie" différents des dérivés non-ioniques de cellulose à substituant(s) hydrophobe(s) de l'invention.

L'agent (ou les agents) d'ajustement de la rhéologie peut (ou peuvent) être choisi(s) parmi les agents épaississants minéraux ou organiques, et en particulier les épaississants associatifs polymériques, les alcools gras différents de ceux de la présente invention tels que l'alcool oléïque, les dérivés cellulosiques autres que les dérivés de cellulose non-ioniques à substituant(s) hydrophobe(s) (A) selon l'invention (hydroxyéthylcellulose, hydroxypropylcellulose, carboxyméthylcellulose) et les gommes d'origine microbienne (gomme de xanthane, gomme de scléroglucane).

La concentration en agent(s) épaississant(s) additionnel(s) est comprise de préférence entre 0,01 et 20% en poids, et de manière plus préférée entre 1 et 10% en poids, par rapport au poids total de la composition

La composition tinctoriale conforme à l'invention peut également contenir un ou plusieurs adjuvant(s) utilisé(s) classiquement dans les compositions pour la teinture des cheveux.

Par «adjuvant(s)», on entend un (ou des) additif(s), différent(s) des composés précités, tels que les agents tensioactifs anioniques, cationiques, non-ioniques, amphotères, zwittérioniques ou leurs mélanges ; les polymères non-ioniques, amphotères, zwittérioniques, anioniques, autres que les dérivés de cellulose non-ioniques à substituant(s) hydrophobe(s) (A) selon l'invention, ou leurs mélanges ; les agents de pénétration ; les agents séquestrants ; les parfums ; les tampons ; les agents dispersants ; les agents conditionneurs tels que par exemple des silicones volatiles ou non volatiles, modifiées ou non modifiées ; les agents filmogènes ; les céramides ; les agents conservateurs ; les agents opacifiants ; les vitamines ; les acides aminés ; les oligopeptides ; les peptides ; les protéines hydrolysées ou non, modifiées ou non ; les enzymes ; les acides et alcools gras ramifiés ou non ; les cires animales, végétales ou minérales ; les acides organiques hydroxylés ; les filtres UV ; les agents anti-oxydants et les agents anti-radicaux libres ; les agents antipelliculaires ; les agents régulateurs de séborrhée ; les agents apaisants ; les huiles minérales, végétales ou animales ; les polyisobutènes et poly($\alpha$-oléfines) ; les pigments ; les acides, bases, plastifiants, charges minérales, nacres, paillettes ; les agents antistatiques et les agents réducteurs.

Le (ou les) adjuvant(s) ci-dessus est (ou sont), en général, présent(s) en quantité comprise, pour chacun d'eux, de préférence entre 0,01 et 40% en poids, et de manière plus préférée entre 0,1 et 25% en poids par rapport au poids de la composition.

Bien entendu, l'homme de l'art veillera à choisir ce (ou ces) éventuel(s) composé(s) complémentaire(s) de manière telle que les propriétés avantageuses attachées intrinsèquement à la composition de teinture d'oxydation conforme à l'invention ne soient pas, ou substantiellement pas, altérées par la (ou les) adjonction(s) envisagée(s).

Le pH de la composition tinctoriale conforme à l'invention va généralement de 3 à 12 environ, et de préférence de 5 à 11 environ. Il peut être ajusté à la valeur désirée au moyen d'agent(s) acidifiant(s) ou alcalinisant(s), habituellement utilisés en teinture des fibres kératiniques ou bien encore à l'aide de système(s) tampon(s) classique(s).

Parmi les agents acidifiants, on peut citer, à titre d'exemple, les acides minéraux ou organiques comme l'acide chlorhydrique, l'acide orthophosphorique, l'acide sulfurique, les acides sulfoniques et les acides carboxyliques comme l'acide acétique, l'acide tartrique, l'acide citrique et l'acide lactique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante :

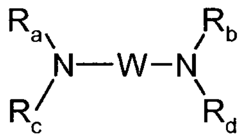

dans laquelle :

- W est un reste propylène éventuellement substitué par un groupe hydroxyle

ou un groupe alkyle en $C_1$-$C_4$ ;

- Ra, Rb, Rc et Rd, identiques ou différents, représentent un atome d'hydrogène, un groupe alkyle en $C_1$-$C_4$ ou hydroxyalkyle en $C_1$-$C_4$.

La composition tinctoriale selon l'invention peut se présenter sous des formes diverses, telles que sous forme de crèmes, de gels, ou sous toute autre forme appropriée pour réaliser une teinture des fibres kératiniques, et notamment des cheveux humains.

Le procédé de teinture des fibres kératiniques de la présente invention est un procédé dans lequel on applique sur les fibres la composition selon la présente invention telle que définie précédemment, de préférence en présence d'au moins un agent oxydant, pendant un temps suffisant pour développer la couleur désirée. La couleur peut être révélée à pH acide, neutre ou alcalin et l'agent (ou les agents) oxydant(s) peut (ou peuvent) être ajouté(s) à la composition de l'invention juste au moment de l'emploi ou il(s) peut (ou peuvent) être mis en oeuvre à partir d'une composition oxydante le(s) contenant, appliquée simultanément ou séquentiellement à la composition de l'invention.

Selon un mode de réalisation particulier, la composition selon la présente invention est une composition prête à l'emploi, mélangée, de préférence au moment de l'emploi, à une composition contenant, dans un milieu approprié pour la teinture, au moins un agent oxydant, cet agent (ou ces agents) oxydant(s) étant présent(s) en une quantité suffisante pour développer une coloration. Le mélange obtenu est ensuite appliqué sur les fibres kératiniques. Après un temps de pause de 3 à 50 minutes environ, de préférence, 5 à 30 minutes environ, les fibres kératiniques sont rincées, lavées au shampooing, rincées à nouveau, puis séchées.

Les agents oxydants classiquement utilisés pour la teinture d'oxydation des fibres kératiniques sont, par exemple, le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, ces oxydoréductases étant éventuellement associées à leurs cofacteurs habituels tels que l'acide urique pour les uricases. L'agent oxydant préféré est le peroxyde d'hydrogène.

La composition oxydante peut également renfermer divers adjuvants utilisés classiquement dans les compositions pour la teinture des cheveux, tels que définis précédemment.

Le pH de la composition oxydante renfermant l'agent oxydant est tel qu'après mélange avec la composition tinctoriale, le pH de la composition résultante appliquée sur les fibres kératiniques varie, de préférence, de 3 à 12 environ, et préférentiellement, de 5 à 10. Il peut être ajusté à la valeur désirée au moyen d'agent(s) acidifiant(s) ou alcalinisant(s) habituellement utilisé(s) en teinture des fibres kératiniques, tel(s) que défini(s) précédemment.

La composition prête à l'emploi qui est finalement appliquée sur les fibres kératiniques, peut se présenter sous des formes diverses, telles que sous forme de crèmes, de gels ou sous toute autre forme appropriée pour réaliser une teinture des fibres kératiniques, et notamment des fibres kératiniques humaines tels que les cheveux.

L'invention a aussi pour objet un dispositif à plusieurs compartiments ou "kit" de teinture comprenant au moins un premier compartiment contenant la composition tinctoriale définie ci dessus et au moins un deuxième compartiment contenant une composition oxydante. Ce dispositif peut être équipé d'un moyen permettant de délivrer sur les cheveux le mélange souhaité, tel que les dispositifs décrits dans la demande de brevet FR A 2 586 913.

Les exemples qui suivent servent à illustrer l'invention sans toutefois présenter un caractère limitatif.

**Exemples**

**<u>Composition 1</u>**

Les compositions suivantes ont été réalisées, les quantités indiquées sont en gramme sauf indication différente.

| | |
|---|---|
| RESORCINOL | 0,685 |
| Monoéthanolam ide d'acide stéarique | 4,8 |
| Acide oleique | 3 |
| CETYL HYDROXYETHYLCELLULOSE ( Natrosol plus grade 330CS commercialisé par Hercules | 0,4 |
| Ammoniaque à 20% de NH3 | 5 |
| DIOXI DE de titane | 0,3 |
| ETHANOLAMI NE | 0,8 |
| OLETH-10 | 1,8 |
| Solution aqueuse à 40 % en poids de polyquaternium-6(chlorure de polydiméthyldiallylammonium, Merquat 100 Commercialisé par Ondeo/Nalco) | 1,6 |
| m-AMI NOPHENOL | 0,14 |
| EDTA | 0,2 |
| 2,4-DIAMINOPHENOXYETHANOL HCl | 0,02 |
| Solution aqueuse à 60 % en poids de chlorure d'hexadiméthrine (Mexomère PO commercialisé par la société Chimex) | 1,2 |
| réducteur | qs |
| antioxydant | qs |
| HYDROXYPROPYL METHYLCELLULOSE | 0,191 |
| OLETH-30 | 1,5 |
| STEARETH-2 | 5,5 |
| Alcools en C20-C22(Nafol 2022 EN commercialisé par la société Sasol) | 3 |
| TOLUENE-2,5-DIAMI NE | 0,7623 |
| eau | qs |

**Protocole d'application**

Chaque composition est mélangée extemporanément, avec une fois et demie son poids avec une composition oxydante de pH voisin de 3 (eau oxygénée à 20 volumes) (6% en poids d'$H_2O_2$). Le mélange se fait facilement et présente une bonne viscosité ; il est appliqué facilement sur des cheveux gris, à 90 % de cheveux blancs, à raison de 10 g pour 1 g de cheveux, pendant 30 minutes. Les cheveux sont ensuite rincés, lavés avec un shampooing standard et séchés.
La coloration capillaire est évaluée de manière visuelle. On obtient ainsi une coloration des cheveux dans une nuance châtain à reflet rouge acajou.
Ces colorations possèdent de bonnes propriétés notamment en termes de sélectivité et d'intensité. Les compositions obtenues sont stables dans le temps.

**Composition 2**

Les compositions suivantes ont été réalisées, les quantités indiquées sont en gramme sauf indication différente.

| | |
|---|---|
| Monoéthanolam ide d'acide stéarique | 4,8 |
| Acide oleique | 3 |
| CETYL HYDROXYETHYLCELLULOSE tel que polysurf 67, hercules | 0.5 |
| p-AMINOPHENOL | 0,24 |
| AMMONIAQUE à 20% de NH3 | 5 |

(suite)

| | |
|---|---|
| DIOXIDE de titane | 0,3 |
| ETHANOLAMINE | 0,8 |
| 2-AMINO-3-HYDROXYPYRIDINE | 0,2 |
| 6-HYDROXYINDOLE | 0,01 |
| Solution aqueuse à 40 % en poids de chlorure de polydiméthyldiallylammonium (Merquat 100 Commercialisé par Ondeo/Nalco) | 1,6 |
| 4-AMI NO-2-HYDROXYTOLUENE | 0,24 |
| 2-METHYL-5-HYDROXYETHYLAMINOPHENOL | 0,15 |
| m-AMINOPHENOL | 0,024 |
| EDTA | 0,2 |
| 2,4-DIAMINOPHENOXYETHANOLHCl | 0,02 |
| Solution aqueuse à 60 % en poids de chlorure d'hexadiméthrine (Mexomère PO commercialisé par la société Chimex) | 1,2 |
| réducteur | qs |
| antioxydant | qs |
| HYDROXYPROPYL METHYLCELLULOSE | 0,191 |
| OLETH-30 | 1,5 |
| PEG-40 STEARATE | 1,8 |
| STEARETH-2 | 5,5 |
| GLYCERYL LAURYL ETHER | 0,5 |
| Alcools en $C_{20}$-$C_{22}$ (Nafol 2022 EN commercialisé par la société Sasol) | 3 |
| TOLUENE-2,5-DIAMINE | 0,2772 |
| eau | qs |

L'application sur les cheveux est effectuée selon le protocole de l'exemple 1 précédent. On obtient ainsi une coloration des cheveux dans une nuance blond à reflet acajou cuivré.

**Les compositions suivantes ont été préparées** (quantités exprimées en g% de matière active)

| | Composition A (invention) | Composition B |
|---|---|---|
| AMMONIUM HYDROXIDE | 4,115 | 4,115 |
| ERYTHORBIC ACID | 0,5 | 0,5 |
| ETHANOLAMINE | 0,7 | 0,7 |
| EDTA | 0,2 | 0,2 |
| SODIUM SULFITE | 0,5 | 0,5 |
| TITANIUM DIOXYDE | 0,3 | 0,3 |
| 4-AMINO-2-HYDROXYTOLUENE | 0,246 | 0,246 |
| p-PHENYLENEDIAMINE | 0,216 | 0,216 |
| HEXADIMETHRINE CHLORIDE | 1 ,2 MA | 1 ,2MA |

(suite)

| | Composition A (invention) | Composition B |
|---|---|---|
| CETYL HYDROXYETHYLCELLULOSE | 0,45 | |
| POLYURETHANE-16 (pur 6) | | 0.45 |
| Acide oléique | 3 | 3 |
| STEARETH-2 | 5,5 | 5,5 |
| STEARAMIDE MEA (96) (and) ETHANOLAMINE (2) (and) STEARIC ACID (2) | 5 | |
| OLETH-30 | 1,5 | 1,5 |
| WATER | Qs 100 | Qs 100 |

Au moment de l'emploi, chacune des compositions est mélangée avec 1 fois ½ son poids d'oxydant 20 volumes (6% en H2O2). Chacun des mélanges est ensuite appliqué sur des mèches de cheveux naturels à 90% de cheveux blancs (BN) et des mèches de cheveux sensibilisés présentant une solubilité alcaline de 22.9% (SA22 .9), à raison de 15 g de mélange par g de cheveu.
A l'issue d'un temps de pause de 30 min à température ambiante, les cheveux sont rincés, lavés avec un shampooing standard, rincés à nouveau et séchés.

**Viscosité des compositions**

Les mesures de viscosité sont réalisées à l'aide d'un rhéomètre référencé METTLER RM180 Rhéomat, les mesures sont faites à 25°C, vitesse de rotation 200 tours/min au moyen
- mobile M4 pour la crème
- mobile M3 ou M2après mélange avec l'oxydant 20 volumes (6% en H2O2) avec un ratio 1+1.5, au (mesure après 2 min de mélange).

| | A | B |
|---|---|---|
| Viscosité de crème M4 (Cps) | 6600 | 4700 |
| Viscosité du mélange avec l'oxydant M3 ou M2 (Cps) | 500 | 160 |

La composition B est trop fluide en crème et en mélange. La composition A est acceptable en crème, fluide en mélange afin de faciliter l'application.

Mesures colorimétrique

La coloration des mèches est évaluée par des mesures colorimétriques dans le système CIE Lab à l'aide du spectrocolorimètre_Konica Minolta CM-2600d
Dans ce système, L représente l'intensité, plus la valeur de L* est faible, plus la coloration obtenues es intense La chromaticité est mesurée par les valeurs a* et b*, a* représentant l'axe ruge/vert et b* l'axe jaune/bleu

**SELECTIVITE DE LA COLORATION**

La sélectivité de la coloration est la variation de la couleur entre des cheveux naturels et des cheveux permanentés Les cheveux naturels sont représentatif de la nature des cheveux à la racine alors que les cheveux sensibilisés sont représentatifs de la nature des cheveux à la pointe.
La sélectivité est mesurée par :

ΔE, qui est la variation de la couleur entre les cheveux naturels et les cheveux permanents, est obtenu à partir de la formule :

$$\Delta E = \sqrt{(L^* - L_o{}^*)^2 + (a^* - a_o{}^*)^2 + (b^* - b_o{}^*)^2}$$

dans laquelle L* représente l'intensité a* et b*, la chromaticité des cheveux colorés naturels et L0* représente l'intensité et a* et b0* la chromaticité des cheveux colorés sensibilisés. Plus la valeur de ΔE est faible, plus la sélectivité est faible et la coloration uniforme le long des cheveux.

Les résultats sont reportés dans le tableau ci-dessous

| | Type chvx | L* | a* | b* | ΔE |
|---|---|---|---|---|---|
| A (invention) | BN | 25,80 | 16,24 | 0,57 | 1,84 |
| | SA22.9 | 24,43 | 17,05 | 1,5 | |
| B (comparatif) | BN | 26,08 | 16,87 | -1,10 | 5,01 |
| | SA22.9 | 21,91 | 15,81 | 1,47 | |

La sélectivité (ΔE) est notablement plus faible pour la composition A selon l'invention par rapport à la composition B ce qui est représentatif d'une coloration plus uniforme le long de la fibre kératine de la pointe à la racine.

## Revendications

**1.** Composition tinctoriale pour fibres kératiniques, comprenant, dans un milieu approprié pour la teinture :

(A) un ou plusieurs dérivé(s) non ionique(s) de cellulose comprenant un ou plusieurs substituant(s) hydrophobe(s) comprenant de 8 à 30 atomes de carbone,
(B) un ou plusieurs colorants d'oxydation, et
(C) un ou plusieurs polymères cationiques diammonium quaternaires contenant des motifs récurrents répondant à la formule (I) :

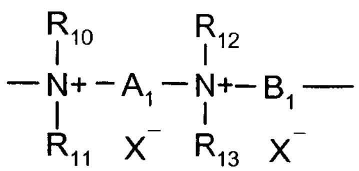

dans laquelle :

$R_{10}$, $R_{11}$, $R_{12}$ et $R_{13}$, identiques ou différents, représentent des radicaux hydrocarbonés, linéaires, ramifiés ou cycliques, saturés, insaturés ou aromatiques, en $C_1$-$C_{20}$, des radicaux hydroxyalkyl linéaires ou ramifiés, dont la partie alkyle est en $C_1$-$C_4$, des radicaux alkyle en $C_1$-$C_6$ linéaires ou ramifiés, substitués par un groupement nitrile, ester, acyle, amide ou -CO-O-$R_{14}$-D ou -CO-NH-$R_{14}$-D avec $R_{14}$ représentant un radical alkyle et D un groupement ammonium quaternaire, ou forment ensemble ou séparément, avec les atomes d'azote auxquels ils sont rattachés, des hétérocycles contenant éventuellement un second hétéroatome autre que l'azote ;
$A_1$ et $B_1$ représentent des radicaux linéaires ou non, saturés ou non, en $C_2$-$C_{20}$,
éventuellement substitués ou interrompus par un ou plusieurs cycles aromatiques, atomes d'oxygène, de soufre ou des groupements portant au moins un de ces atomes ;
X- désigne un anion organique ou minéral ;
$A_1$, $R_{10}$ et $R_{12}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, $B_1$ peut également désigner un groupement $-(CH_2)_n-CO-D-OC-(CH_2)_n-$ dans lequel n est compris entre 1 et 100, et D désigne un reste glycol, diamine bis-secondaire, diamine bis-primaire, uréylène.

**2.** Composition tinctoriale selon la revendication 1, **caractérisée en ce que** le dérivé non ionique de cellulose (A) est

une hydroxyéthylcellulose substituée par un ou plusieurs substituant(s) hydrophobe(s) comprenant de 8 à 30 atomes de carbone.

3. Composition tinctoriale selon l'une quelconque des revendications précédentes, **caractérisée en ce que** le substituant hydrophobe est un groupe alkyle en $C_{10}$-$C_{22}$.

4. Composition tinctoriale selon l'une quelconque des revendications précédentes, **caractérisée en ce que** le substituant hydrophobe est un groupement cétyle.

5. Composition tinctoriale selon l'une quelconque des revendications précédentes, **caractérisée en ce que** le degré de substitution hydrophobe va de 0,1 à 10% en poids, de préférence de 0,1 à 1% en poids et de manière plus préférée de 0,4 à 0,8% en poids, du poids total du polymère.

6. Composition tinctoriale selon l'une quelconque des revendications précédentes, **caractérisée en ce que** la concentration en dérivé(s) non-ionique(s) de cellulose (A) va de 0,01 à 10% en poids, de préférence de 0,05 à 3% en poids et de manière plus préférée de 0,1 à 1% en poids, par rapport au poids total de la composition.

7. Composition tinctoriale selon l'une quelconque des revendications précédentes, **caractérisée en ce que** le colorant d'oxydation (B) est choisi parmi les bases d'oxydation, les coupleurs d'oxydation, et leurs sels d'addition.

8. Composition tinctoriale selon la revendication précédente, **caractérisée en ce que** le colorant d'oxydation est une base d'oxydation choisie parmi les bases d'oxydation para-phénylènediamines, les bis-phénylalkylènediamines, les para-aminophénols, les bis-para-aminophénols, les ortho-aminophénols, les bases hétérocycliques et leurs sels d'addition.

9. Composition tinctoriale selon l'une quelconque des revendications précédentes **caractérisée en ce que** le colorant d'oxydation est un coupleur d'oxydation choisi parmi les coupleurs benzéniques, les coupleurs hétérocycliques, les coupleurs naphtaléniques, et leurs sels d'addition.

10. Composition tinctoriale selon la revendication précédente, **caractérisée en ce que** le coupleur benzénique est choisi parmi les méta-aminophénols, les méta-phénylènediamines, les méta-diphénols, et leurs sels d'addition.

11. Composition tinctoriale selon l'une quelconque des revendications précédentes, **caractérisée en ce que** la concentration en coupleur(s) d'oxydation va de 0,001 à 20 % en poids, de préférence de 0,005 à 10 % en poids, et encore plus préférentiellement de 0,01 à 5 % en poids, par rapport au poids total de la composition.

12. Composition selon l'une quelconque des revendications précédentes dans laquelle le polymère cationique présente une densité de charge d'au moins 1 meq/g.

13. Composition selon la revendication précédente, **caractérisée en ce que** le polymère cationique est choisi parmi les polymères aux motifs récurrents de formules (W) et (U) suivantes :

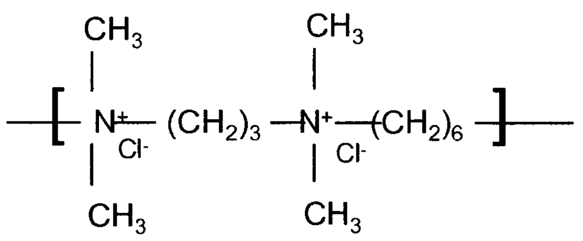

**(W)**

et

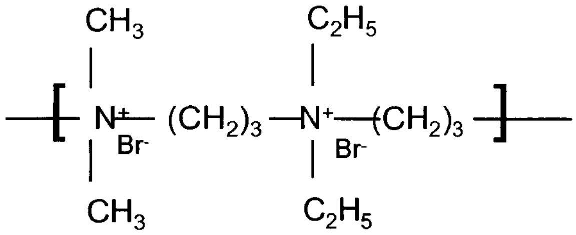

**(U)**

**14.** Composition selon l'une des revendications précédentes, **caractérisée en ce que** la teneur en polymère cationique représente 0,01 à 10 % en poids par rapport au poids de la composition, plus particulièrement 0,05 et 6 % en poids rapport au poids de la composition.

**15.** Composition tinctoriale selon l'une quelconque des revendications précédentes, **caractérisée en ce qu'**elle comprend un ou plusieurs colorant(s) direct(s) choisi(s) parmi les colorants nitrés benzéniques, les colorants directs azoïques, les colorants directs méthiniques, les colorants anthraquinoniques, les colorants xanthéniques, les colorants triarylméthaniques et leurs sels d'addition.

**16.** Composition selon l'une quelconque des revendications précédentes comprenant une ou plusieurs alcanolamides.

**17.** Composition tinctoriale selon l'une quelconque des revendications précédentes, **caractérisée en ce qu'**elle comprend au moins un agent oxydant.

**18.** Procédé de teinture d'oxydation des fibres kératiniques, **caractérisé en ce qu'**on applique sur les fibres une composition tinctoriale, telle que définie dans l'une quelconque des revendications 1 à 16 en présence d'au moins un agent oxydant, pendant un temps suffisant pour développer la couleur désirée.

**19.** Dispositif à plusieurs compartiments, **caractérisé en ce qu'**il comprend au moins un premier compartiment contenant une composition tinctoriale telle que définie dans l'une quelconque des revendications 1 à 16 et au moins un deuxième compartiment contenant au moins un agent oxydant.

## Patentansprüche

**1.** Färbezusammensetzung für Keratinfasern, die in einem für die Färbung geeigneten Medium Folgendes umfasst:

(A) ein oder mehrere nichtionische Cellulosederivate mit einem oder mehreren hydrophoben Substituenten mit 8 bis 30 Kohlenstoffatomen,
(B) einen oder mehrere Oxidationsfarbstoffe und
(C) ein oder mehrere kationische quaternäre Diammoniumpolymere mit Wiederholungseinheiten der Formel (I):

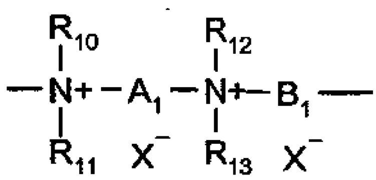

worin:

$R_{10}$, $R_{11}$, $R_{12}$ und $R_{13}$ gleich oder verschieden sind und für lineare, verzweigte oder cyclische, gesättigte, ungesättigte oder aromatische $C_1$-$C_{20}$-Kohlenwasserstoffreste, lineare oder verzweigte Hydroxyalkylreste, deren Alkylteil $C_1$-$C_4$ ist, lineare oder verzweigte $C_1$-$C_6$-Alkylreste, die durch eine Nitril-, Ester-, Acyl-, Amid- oder -CO-O-$R_{14}$-D- oder -CO-NH-$R_{14}$-D-Gruppe substituiert sind,
wobei $R_{14}$ für einen Alkylrest steht und D für eine quaternäre Ammoniumgruppe steht, stehen oder gemeinsam oder getrennt mit den Stickstoffatomen, an die sie gebunden sind, Heterocyclen, die gegebenenfalls

ein zweites Heteroatom, das von Stickstoff verschieden ist, enthalten, bilden;
$A_1$ und $B_1$ für lineare oder nichtlineare, gesättigte oder ungesättigte $C_2$-$C_{20}$-Reste, die gegebenenfalls durch einen oder mehrere aromatische Ringe, ein oder mehrere Sauerstoff- oder Schwefelatome oder Gruppen, die mindestens eines dieser Atome tragen, substituiert oder unterbrochen sind;
$X^-$ für ein organisches oder anorganisches Anion steht;
$A_1$, $R_{10}$ und $R_{12}$ mit den beiden Stickstoffatomen, an die sie gebunden sind, einen Piperazinring bilden können; $B_1$ außerdem dann, wenn $A_1$ für einen linearen oder verzweigten, gesättigten oder ungesättigten Alkylen- oder Hydroxyalkylenrest steht, auch für eine - $-(CH_2)_n$-CO-D-OC-$(CH_2)_n$-Gruppe stehen kann, worin n zwischen 1 und 100 liegt und
D für einen Glykolrest, einen bissekundären Diaminrest, einen bisprimären Diaminrest oder einen Ureylenrest steht.

2. Färbezusammensetzung nach Anspruch 1, **dadurch gekennzeichnet, dass** es sich bei dem nichtionischen Cellulosederivat (A) um eine durch einen oder mehrere hydrophobe Substituenten mit 8 bis 30 Kohlenstoffatomen substituierte Hydroxyethyl-cellulose handelt.

3. Färbezusammensetzung nach einem der vorhergehenden Ansprüche, **dadurch gekennzeichnet, dass** es sich bei dem hydrophoben Substituenten um eine $U_{10}$-$C_{22}$-Alkylgruppe handelt.

4. Färbezusammensetzung nach einem der vorhergehenden Ansprüche, **dadurch gekennzeichnet, dass** es sich bei dem hydrophoben Substituenten um eine Cetylgruppe handelt.

5. Färbezusammensetzung nach einem der vorhergehenden Ansprüche, **dadurch gekennzeichnet, dass** der Grad der hydrophoben Substitution 0,1 bis 10 Gew.-%, vorzugsweise 0,1 bis 1 Gew.-% und weiter bevorzugt 0,4 bis 0,8 Gew.-%, bezogen auf das Gesamtgewicht des Polymers, beträgt.

6. Färbezusammensetzung nach einem der vorhergehenden Ansprüche, **dadurch gekennzeichnet, dass** die Konzentration an nichtionischem Cellulosederivat bzw. nichtionischen Cellulosederivaten (A) 0,01 bis 10 Gew.-%, vorzugsweise 0,05 bis 3 Gew.-% und weiter bevorzugt 0,1 bis 1 Gew.-%, bezogen auf das Gesamtgewicht der Zusammensetzung, beträgt.

7. Färbezusammensetzung nach einem der vorhergehenden Ansprüche, **dadurch gekennzeichnet, dass** der Oxidationsfarbstoff (B) aus Oxidationsbasen, Oxidationskupplern und Additionssalzen davon ausgewählt ist.

8. Färbezusammensetzung nach dem vorhergehenden Anspruch, **dadurch gekennzeichnet, dass** es sich bei dem Oxidationsfarbstoff um eine Oxidationsbase, die aus para-Phenylendiamin-Oxidationsbasen, Bisphenylalkylendiaminen, para-Aminophenolen, Bis-para-aminophenolen, ortho-Aminophenolen, heterocyclischen Basen und Additionssalzen davon ausgewählt ist, handelt.

9. Färbezusammensetzung nach einem der vorhergehenden Ansprüche, **dadurch gekennzeichnet, dass** es sich bei dem Oxidationsfarbstoff um einen Oxidationskuppler, der aus Benzol-Kupplern, heterocyclischen Kupplern, Naphthalin-Kupplern und Additionssalzen davon ausgewählt ist, handelt.

10. Färbezusammensetzung nach dem vorhergehenden Anspruch, **dadurch gekennzeichnet, dass** der Benzol-Kuppler aus meta-Aminophenolen, meta-Phenylendiaminen, meta-Diphenolen und Additionssalzen davon ausgewählt ist.

11. Färbezusammensetzung nach einem der vorhergehenden Ansprüche, **dadurch gekennzeichnet, dass** die Konzentration an Oxidationskuppler(n) im Bereich von 0,001 bis 20 Gew.-%, vorzugsweise 0,005 bis 10 Gew.-% und noch weiter bevorzugt 0,01 bis 5 Gew.-%, bezogen auf das Gesamtgewicht der Zusammensetzung, liegt.

12. Zusammensetzung nach einem der vorhergehenden Ansprüche, in der das kationische Polymer in einer Ladungsdichte von mindestens 1 meq/g vorliegt.

13. Zusammensetzung nach dem vorhergehenden Anspruch, **dadurch gekennzeichnet, dass** das kationische Polymer aus Polymeren mit Wiederholungseinheiten der folgenden Formeln (W) und (U) ausgewählt ist:

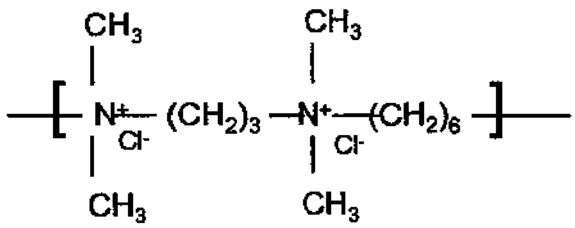

(W)

und

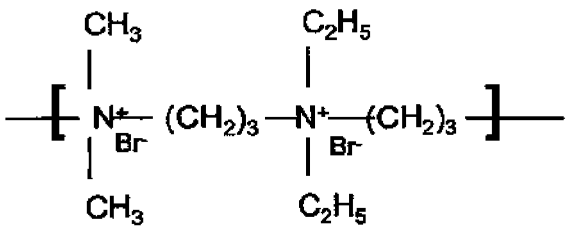

(U)

**14.** Zusammensetzung nach einem der vorhergehenden Ansprüche, **dadurch gekennzeichnet, dass** der Gehalt an kationischem Polymer 0,01 bis 10 Gew.-%, bezogen auf das Gewicht der Zusammensetzung, spezieller 0,05 bis 6 Gew.-%, bezogen auf das Gewicht der Zusammensetzung, beträgt.

**15.** Färbezusammensetzung nach einem der vorhergehenden Ansprüche, **dadurch gekennzeichnet, dass** sie einen oder mehrere Direktfarbstoffe, die aus Nitrobenzol-Farbstoffen, Azo-Direktfarbstoffen, Methin-Direktfarbstoffen, Anthrachinon-Farbstoffen, Xanthen-Farbstoffen, Triarylmethan-Farbstoffen und Additionssalzen davon ausgewählt sind, umfasst.

**16.** Zusammensetzung nach einem der vorhergehenden Ansprüche, die ein oder mehrere Alkanolamine umfasst.

**17.** Färbezusammensetzung nach einem der vorhergehenden Ansprüche, **dadurch gekennzeichnet, dass** sie mindestens ein Oxidationsmittel umfasst.

**18.** Verfahren zum Oxidationsfärben von Keratinfasern, **dadurch gekennzeichnet, dass** man auf die Fasern eine Färbezusammensetzung gemäß einem der Ansprüche 1 bis 16 in Gegenwart mindestens eines Oxidationsmittels über einen zur Entwicklung der gewünschten Färbung ausreichenden Zeitraum aufbringt.

**19.** Vorrichtung mit mehreren Kompartimenten, **dadurch gekennzeichnet, dass** sie mindestens ein erstes Kompartiment, das eine Färbezusammensetzung gemäß einem der Ansprüche 1 bis 16 enthält, und mindestens ein zweites Kompartiment, das mindestens ein Oxidationsmittel enthält, umfasst.

## Claims

**1.** Dye composition for keratin fibres, comprising, in a medium suitable for dyeing:

(A) one or more nonionic derivative(s) of cellulose comprising one or more hydrophobic substituent(s) containing from 8 to 30 carbon atoms;
(B) one or more oxidation dyes, and
(C) one or more diquaternary ammonium cationic polymers comprising repeat units corresponding to formula (I):

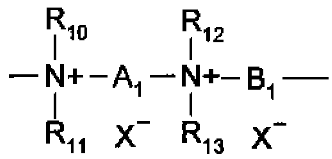

in which:

$R_{10}$, $R_{11}$, $R_{12}$ and $R_{13}$, which may be identical or different, represent linear, branched or cyclic, saturated, unsaturated or aromatic $C_1$-$C_{20}$ hydrocarbon-based radicals, linear or branched hydroxyalkyl radicals in which the alkyl part is $C_1$-$C_4$, or linear or branched $C_1$-$C_6$ alkyl radicals substituted with a nitrile, ester, acyl, amide, -CO-O-$R_{14}$-D or -CO-NH-$R_{14}$-D group with $R_{14}$ representing an alkyl radical and D representing a quaternary ammonium group, or, together or separately, form, with the nitrogen atoms to which they are attached, heterocycles optionally containing a second heteroatom other than nitrogen;

$A_1$ and $B_1$ represent linear or nonlinear, saturated or unsaturated $C_2$-$C_{20}$ radicals optionally substituted or interrupted with one or more aromatic rings, oxygen or sulphur atoms or groups bearing at least one of these atoms;

$X^-$ denotes an organic or mineral anion;

$A_1$, $R_{10}$ and $R_{12}$ can form, with the two nitrogen atoms to which they are attached, a piperazine ring; in addition, if $A_1$ denotes a linear or branched, saturated or unsaturated alkylene or hydroxyalkylene radical, $B_1$ may also denote a $-(CH_2)_n$-CO-D-OC-$(CH_2)_n$- group in which n is between 1 and 100, and D denotes a glycol, bissecondary diamine, bisprimary diamine or ureylene residue.

**2.** Dye composition according to Claim 1, **characterized in that** the nonionic derivative of cellulose (A) is a hydroxyethylcellulose substituted with one or more hydrophobic substituent(s) containing from 8 to 30 carbon atoms.

**3.** Dye composition according to either one of the preceding claims, **characterized in that** the hydrophobic substituent is a $C_{10}$-$C_{22}$ alkyl group.

**4.** Dye composition according to any one of the preceding claims, **characterized in that** the hydrophobic substituent is a cetyl group.

**5.** Dye composition according to any one of the preceding claims, **characterized in that** the degree of hydrophobic substitution ranges from 0.1% to 10% by weight, preferably from 0.1% to 1% by weight, and more preferably from 0.4% to 0.8% by weight, of the total weight of the polymer.

**6.** Dye composition according to any one of the preceding claims, **characterized in that** the concentration of nonionic derivative(s) of cellulose (A) ranges from 0.01% to 10% by weight, preferably from 0.05% to 3% by weight, and more preferably from 0.1% to 1% by weight, relative to the total weight of the composition.

**7.** Dye composition according to any one of the preceding claims, **characterized in that** the oxidation dye (B) is chosen from oxidation bases and oxidation couplers, and addition salts thereof.

**8.** Dye composition according to the preceding claim, **characterized in that** the oxidation dye is an oxidation base chosen from the oxidation bases: para-phenylenediamines, bisphenylalkylenediamines, para-aminophenols, les bis-para-aminophenols, ortho-aminophenols and heterocyclic bases, and addition salts thereof.

**9.** Dye composition according to any one of the preceding claims, **characterized in that** the oxidation dye is an oxidation coupler chosen from benzene couplers, heterocyclic couplers and naphthalene couplers, and addition salts thereof.

**10.** Dye composition according to the preceding claim, **characterized in that** the benzene coupler is chosen from meta-aminophenols, meta-phenylenediamines and meta-diphenols, and addition salts thereof.

**11.** Dye composition according to any one of the preceding claims, **characterized in that** the concentration of oxidation coupler(s) ranges from 0.001% to 20% by weight, preferably from 0.005% to 10% by weight, and even more preferably from 0.01% to 5% by weight, relative to the total weight of the composition.

**12.** Composition according to any one of the preceding claims, in which the cationic polymer has a charge density of at least 1 meq/g.

**13.** Composition according to the preceding claim, **characterized in that** the cationic polymer is chosen from polymers with repeat units of formulae (W) and (U) below:

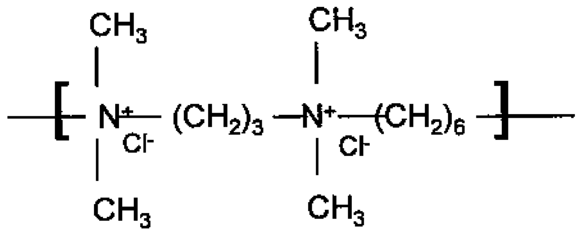

**(W)**

and

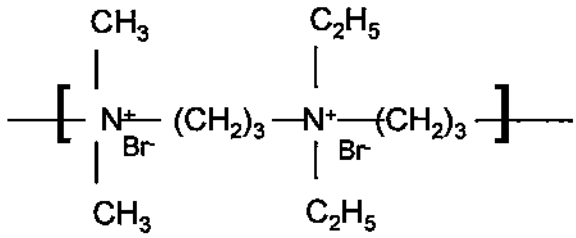

**(U)**

**14.** Composition according to one of the preceding claims, **characterized in that** the content of cationic polymer represents 0.01% to 10% by weight, relative to the weight of the composition, more particularly 0.05% to 6% by weight, relative to the weight of the composition.

**15.** Dye composition according to any one of the preceding claims, **characterized in that** it comprises one or more direct dye(s) chosen from nitrobenzene dyes, azo direct dyes, methine direct dyes, anthraquinone dyes, xanthene dyes and triarylmethane dyes, and addition salts thereof.

**16.** Composition according to any one of the preceding claims, comprising one or more alkanolamides.

**17.** Dye composition according to any one of the preceding claims, **characterized in that** it comprises at least one oxidizing agent.

**18.** Process for the oxidation dyeing of keratin fibres, **characterized in that** a dye composition as defined in any one of Claims 1 to 16 is applied to the fibres in the presence of at least one oxidizing agent for a period of time sufficient to develop the desired colour.

**19.** Multicompartment device, **characterized in that** it comprises at least a first compartment containing a dye composition as defined in any one of Claims 1 to 16 and at least a second compartment containing at least one oxidizing agent.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9803150 A
- EP 1707190 A
- GB 1026978 A
- GB 1153196 A
- FR 2801308
- DE 2359399
- JP 63169571 A
- JP 5063124 A
- EP 0770375 A
- WO 9615765 A
- FR 2750048 A
- DE 3843892 A
- DE 4133957 A
- WO 9408969 A
- WO 9408970 A
- FR 2733749 A
- DE 19543988 A
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- FR 2586913 A